# EUROPEAN PATENT SPECIFICATION

(11) **EP 2 756 837 B1**
(45) Date of publication and mention of the grant of the patent: **10.04.2019**
(21) Application number: 13000220.7
(22) Date of filing: 16.01.2013
(51) Int. Cl.: A61K 6/02, A61K 6/00

(54) **TEMPORARY ROOT CANAL SEALER DISPERSION**
DISPERSION ZUR VORÜBERGEHENDEN VERSIEGELUNG EINER ZAHNWURZEL
DISPERSION DE SCELLEMENT DE CANAL RADICULAIRE TEMPORAIRE

(43) Date of publication of application: 23.07.2014
(73) Proprietor: Dentsply DeTrey GmbH, 78467 Konstanz (DE)
(72) Inventor: Walz, Uwe, 78465 Konstanz (DE); Weber, Christoph, Dr., 78464 Konstanz (DE); Brod, Carsten, 78467 Konstanz (DE); Klee, Joachim, E., Prof. Dr., 78315 Radolfzell (DE)
(74) Representative: Hartz, Nikolai

(56) References cited:
- EP-A1- 2 229 929
- FR-A1- 2 684 873
- US-A1- 2002 198 283
- DATABASE EPODOC [Online] EUROPEAN PATENT OFFICE, THE HAGUE, NL; 30 September 1997 (1997-09-30), Nagata Satoshi et al.: "Dental root canal filler composition", XP002698340, Database accession no. JP-33238195-A & JP H09 169612 A (NIPPON SHIKA YAKUHIN KK) 30 June 1997 (1997-06-30) -& DATABASE WPI Week 199736 Thomson Scientific, London, GB; AN 1997-389318 XP002699873, & JP H09 169612 A (NIPPON SHIKA YAKUHIN KK) 30 June 1997 (1997-06-30)
- DATABASE WPI Week 200324 Thomson Scientific, London, GB; AN 2003-245962 XP002698342, & KR 2002 0078253 A (METABIOMED CO LTD) 18 October 2002 (2002-10-18)

## Description

### Field of the Invention

The present invention relates to a temporary root canal sealer composition. Moreover, the present invention also relates to a process for the preparation of a temporary root canal sealer composition. A temporary root canal sealer composition according to the present invention has improuved storage stability and in-une stability as well as excellent radio opacity.

### Background of the Invention

Temporary root canal sealers are known. EP 2229929 discloses a temporary root canal sealer dispersion having a pH of at least 9 comprising (i) an aqueous dispersion medium, comprising an oligomer and/or polymer having Lewis basic groups, and optionally a hydroxide of an alkali metal and/or a hydroxide of an alkaline earth metal, and (ii) a dispersed phase comprising a radio-opaque filler.

EP0464545 discloses a paste composition which comprises an alkaline-earth metal hydroxide, a polyhydric alcohol, an alkali-soluble cellulose derivative, and water.

JP3027309 discloses a root canal filler which comprises (A) a powder agent, comprising calcium phosphate powder consisting of alpha-tricalcium phosphate and of monocalcium phosphate and iodoform powder and (B) a curing solution comprising an aqueous solution of a polyalkylene glycol.

A simple paste obtained by mixing calcium hydroxide with polyethylene glycol 400 is disclosed by Zelante et al. (Revista de Odontologia da UNESP 21, 37-46 (1992)).

Ulyssea et al. (Revista da Faculdade de Odontologia de Pelotas 213, 38-41 (1992)) discloses the use of barium sulphate as the radio opacifier in a 1:4 ratio with the calcium hydroxide powder.

Maeda T. (Journal of the Osaka University Dental Society 5, 57-62, (1960)) discloses a paste containing calcium hydroxide and polyethylene glycol 1500 as a base, and sulphisomidine and eugenol as antibacterial agents.

Leonardo et al. (Revista da Faculdade de Farmacia e Odontologia de Araraquara, 10(suppl. I), 125-35 (1976)) discloses a paste containing calcium hydroxide, polyethyleneglycol 400, barium sulphate for radioopacity and hydrogenised colophony to improve physical properties.

Temporary root canal sealers are used for temporarily obturating a root canal cavity formed upon extraction of dental pulp and/or root canal tissue. The temporary root canal sealer composition solidifies in the root canal usually by drying. Since the solidified temporary root canal sealer composition is eventually removed from the root canal and replaced by a permanent obturation, easy removal of the temporary root canal sealer from the root canal by dissolving the solidified sealer composition is essential. Accordingly, the components of a temporary root canal sealer compositions should not undergo chemical crosslinking reactions.

Conventional temporary root canal sealer compositions comprise a strongly alkaline aqueous slurry of calcium hydroxide and a radio-opaque filler such as barium sulfate. Given that radioopacity depends on the atom number of the elements contained in the filler, desirable improvements regarding radio opaque fillers usually lead to high density materials. As a consequence, sedimentation of the radio opaque filler may occur, which leads to a storage stability problem of the temporary root canal composition.

Moreover, the strongly alkaline medium present in a root canal sealer composition may deteriorate components in the composition due to hydrolysis or condensation reactions as well as other reaction pathways. Accordingly, the storage stability problem is further aggravated due the harsh pH conditions present in a temporary root canal sealer composition.

On the other hand, when a package of the temporary root canal sealer composition is opened, it is desirable that the content of the package remains useful for an extended period of time, preferably throughout the entire workday so that different patients may be treated with a single package. Therefore, in case a temporary root canal sealer composition solidifies too quickly once the package wherein the composition was stored, is opened, an in-use stability problem arises.

### Summary of the Invention

It is the problem of the present invention to provide a temporary root canal sealer composition which has high radio-opacity, high storage stability and high in-use stability, preferably of at least 6 hours.

It is also a problem of the present invention to provide a process for preparing a temporary root canal sealer composition, which may be used on an industrial scale for providing a temporary root canal sealer composition which has high radio-opacity, high storage stability and high in-use stability, preferably of at least 6 hours.

These problems are solved according to the present invention by temporary root canal sealer composition, comprising based on the total weight of the composition:
(a) 0.5 to 40 wt% of a water-soluble polymer having an average molecular weight of from 5000 to 100,000;
(b) 20 to 40 wt% of a particulate inorganic radio opaque filler;
(c) 0.01 to 5 wt.% of an alkali resistant particulate filler having an average particle size of from 1 to 100 nm;
(d) 5 to 25 wt% of an alkali earth metal hydroxide and/or alkali metal hydroxide;
(e) 15 to 45 wt% of water; and
(f) 0 to 25 wt% of a humectant.

The present invention additionally provides a process for the preparation of a temporary root comprising
(A) a step of mixing:
   (i) 0.5 to 40 wt% of a water-soluble polymer having an average molecular weight of from 5000 to 100,000;
   (ii) 20 to 40 wt% of a particulate inorganic radio opaque filler;
   (iii) 0.01 to 5 wt.% of an alkali resistant particulate filler having an average particle size of from 1 to 100 nm;
   (iv) 5 to 25 wt% of an alkali earth metal hydroxide and/or alkali metal hydroxide;
   (v) 15 to 45 wt% of water; and
   (vi) 0 to 25 wt% of a humectant.
   in water, and
(B) a step of dispersing the mixture.

The present invention is based on the recognition that a combination of a water-soluble polymer having an average molecular weight of from 5000 to 100,000, and an alkali resistant particulate filler having an average particle size of from 1 to 100 nm provides a stable aqueous dispersion which efficiently stabilizes a dispersion of a particulate inorganic radio opaque filler over a long period of time even when the density of the filler is high such as in case of the calcium tungstate. Moreover, the combination of a water-soluble polymer having an average molecular weight of from 5000 to 100,000, and an alkali resistant particulate filler having an average particle size of from 1 to 100 nm provides an aqueous dispersion which has a high in-use stability of up to 6 hours. The storage stability may be further improved by a specific dispersion method.

### Detailed Description of the Invention

The present invention relates to a temporary root canal sealer composition. The temporary root canal sealer composition of the invention is in the form of a dispersion. In the dispersion, a dispersing phase and a dispersed phase are present. In general, the particulate inorganic radio opaque filler forms part of the dispersed phase.

The properties of a root canal sealer and, in particular, a temporary root canal sealer dispersion depend on many factors, but general trends can be seen between the composition of the aqueous dispersion medium and the temporary root canal sealer dispersion properties. Since the trends are not necessarily linear and there are many interactions, trends should not be extrapolated too far from known points. A change of most components in an aqueous dispersion medium affects the degree to which the dispersed phase may be incorporated therein and, hence, amongst other properties, affects the radio-opacity, viscosity, homogeneity and consistency of the temporary root canal sealer dispersion in some way. The following trends are observed in analysis of the aqueous dispersion medium and the properties of the resulting temporary root canal sealer dispersions.

The temporary root canal sealer composition comprises, based on the total weight of the composition, 0.5 to 40 wt%, preferably 25 to 35 wt%, of a water-soluble polymer having an average molecular weight of from 5000 to 100,000.

The water-soluble polymer is preferably selected from polyethylene glycol, polyvinyl alcohol, or polyvinyl pyrrolidone. The water-soluble polymer may be used as a mixture of two or more polymers. Preferably, the water-soluble polymer is polyethylene glycol. The use of the water-soluble polymer, in particular polyethylene glycol allows the radio-opaque filler to be dispersed in the composition in considerable amounts without affecting the stability of the temporary root-canal sealer composition such that the radio-opaque filler precipitates therefrom.

If a lower weight percent of water-soluble polymer is employed in the composition, the temporary root canal sealer composition is insufficiently plastic as to be effective for sealing and the sealer separates due to the high density of the radio-opaque filler. Moreover, if a lower weight percent of water-soluble polymer is employed in the composition, the consistency and homogeneity of the resulting temporary root canal sealer composition is decreased, such that the composition is likewise rendered unsuitable for use as a root canal sealer. The reduced plasticity, consistency and homogeneity also render such composition ineffective as visualizing agents for use in combination with X-ray radiation.

Conversely, if a greater weight percent of the water-soluble polymer is employed in the temporary root canal sealer composition, the remaining components thereof are effectively diluted, such that the radio-opacity of the dispersion is likewise reduced.

The polyethylene glycol of the temporary root canal sealer has an average molecular weight of from 5000 to 100,000. Preferably, the polyethylene glycol has an average molecular weight of from 6000 to 10,000.

In the event that the average molecular weight of the water-soluble polymer lies above the maximum value specified, the viscosity of the composition is so high that it not only prevents formation of a consistent temporary root canal sealer composition, but also prevents the radio opaque filler from being incorporated therein. In contrast, if the average molecular weight of the water-soluble polymer lies below the minimum value specified, the viscosity of the temporary root canal sealer composition is so low that the radio opaque filler settles out of the composition, particularly when dense radio opaque fillers are employed in an increased weight percent based on the total amount of the composition. Moreover, if the average molecular weight of the water-soluble polymer lies below the minimum value specified, the viscosity of the temporary root canal sealer composition is so low that it prevents the composition from sealing the root canal effectively and/or for sufficient lengths of time. Under such circumstances, the temporary root canal sealer composition is rendered ineffective as a radio-opaque sealer.

The average molecular weight, Mₙ, of the water-soluble polymer may be determined by any method for determining the distribution in molecular weight of a macromolecule. Typical methods include size exclusion chromatographic techniques such as gel permeation chromatography or gel filtration chromatography, as well as mass spectrometric techniques such as electrospray mass spectrometry or matrix-assisted laser desorption/ionisation spectroscopy, and/or viscosity measurements. Mass spectrometric techniques such as electrospray mass spectrometry or matrix-assisted laser desorption/ionisation spectroscopy are particularly preferred for the determination of the average molecular weight, Mₙ, of the water-soluble polymer.

The temporary root canal sealer composition comprises, based on the total weight of the composition, (b) 20 to 40 wt% of a particulate inorganic radio opaque filler. The radio opaque filler may be a finely divided particulate material which is comprised of crystalline, amorphous or metallic fibers, flakes, powders or colloids. Preferably, the radio opaque filler is a finely divided powder. The particles of radio opaque filler are sufficiently finely divided so as to render the temporary root canal sealer composition radio-opaque to X-rays, relative to another material. The radio opaque filler may be present in the temporary root canal sealer composition in the form of a hydrated or non-hydrated solid. In the event that the radio-opaque filler is even slightly soluble in any of the other components of the temporary root canal sealer composition, the radio-opaque filler may also be present therein in the form of a solution.

Preferably, the particulate inorganic filler may be selected from calcium tungstate, barium tungstate and barium sulfate. Preferably, the temporary root canal sealer comprises 25 to 35 wt% of particulate inorganic radio opaque filler, preferably calcium tungstate.

Preferably, the temporary root canal sealer composition comprises particulate inorganic radio opaque filler having an average particle size of from 0.5 to 100 µm, more preferably from 2 to 30 µm.

The temporary root canal sealer composition comprises, based on the total weight of the composition, (c) 0.01 to 5 wt.%, preferably 0.1 to 4 wt.%, of an alkali resistant particulate filler having an average particle size of from 1 to 100 nm. The alkali resistant particulate filler may be selected from zirconium dioxide and titanium dioxide or mixed oxides of zirconium and titanium. Preferably the temporary root canal sealer composition comprises 0.5 to 2 wt% of zirconium dioxide.

The alkali resistant particulate filler is a non-reactive inorganic filler, which does not crosslink under alkaline conditions, thereby forming a gel. The use of alkali resistant particulate filler allows the radio opaque filler to be dispersed in the temporary root canal sealer composition in considerable amounts without affecting the stability of the temporary root-canal sealer composition.

In general, the alkali resistant particulate filler has an average particle size of from 1 to 100 nm. Preferably the alkali resistant particulate filler has an average particle size of from 5 to 50 nm.

The alkali resistant particulate filler can be obtained by hydrolytic condensation of hydrolyzable precursor compounds of zirconium and/or titanium and optionally further hydrolyzable compounds of other metals which are incorporated into the inorganic network during the hydrolysis. Preferred compounds can be represented by the general formula MXₙR¹ₜ, wherein M denotes Ti or Zr; X is a hydrolyzable group R¹, denotes independently, an aliphatic, cycloaliphatic, or aromatic radical with 1 to 30 carbon atoms; n represents an integer of 1-4; and t represents an integer of 0-3.

Specific examples of Zr and Ti compounds are TiCl₄, Ti(OC₂H₅)₄, Ti(OCH₃H₇)₄, Zr(OC₄H₉)₄, ZrCl₄, Zr(OC₂H₅)₄, Zr(OC₃H₇)₄, Zr(OC₄H₉)₄, and ZrOCl₂.

Moreover, boron, tin, and barium compounds can also be incorporated into the hydrolyzable precursor compound mixture. Suitable compounds are, for example, BCl₃, B(OCH₃)₃, B(OC₂H₅)₃, SnCl₄, Sn(OCH₃)₄, Ba(OCH₃)₃, Ba(OC₂H₅)₃, and Ba(OCOCH₃)₂.
By incorporating heavy elements, in particular, Zr, Ti, or Ba into the alkali resistant particulate filler, it is possible to increase the X-ray opacity of the temporary root canal filler composition.

Furthermore, the mixtures from which the alkali resistant particulate filler is produced can contain hydroiyzabie aluminum compounds. The hydrolyzable aluminum compounds can be represented by the general formula AlR²₃, wherein the groups R² can be the same or different and are halogen atoms, alkoxy groups, alkoxycarbonyl groups, alkyl groups, aryl groups, and hydroxy groups. Aluminum alkoxides and aluminum halides are preferred aluminum compounds. Specific examples are Al(OCH₃)₃, Al(OC₂H₅)₃, Al(OC₃H₇)₃, Al(OC₄H₉)₃, AlCl₃, and AlCl(OH)₂.

The production of the inorganic networks can take place in the way which is common in the field of poly(hetero)condensation.

The temporary root canal sealer composition comprises, based on the total weight of the composition, (d) 5 to 25 wt% of an alkali earth metal hydroxide and/or alkali metal hydroxide. The alkali earth metal may be selected from calcium, magnesium or barium. The alkali metal may be selected from lithium, sodium and potassium. The temporary root canal sealer composition may comprise a single hydroxide or a mixture of two or more hydroxides of an alkali earth metal or an alkali metal. It is preferred to use hydroxides of a metal having a high atom number. Accordingly, the temporary root canal sealer composition preferably comprises 10 to 20 wt% of calcium hydroxide.

A hydroxide may be added directly to the composition or it may be generated *in situ* in the aqueous environment from an appropriate precursor. Precursors suitable for the *in situ* generation of the calcium hydroxide comprise calcium oxides (including the peroxides and superoxides), carbonates and bicarbonates, either singly or in combination.

The alkali earth metal hydroxide and/or alkali metal hydroxide is present in the temporary root canal sealer composition in an amount sufficient to provide an elevated pH in the alkaline range. In a preferred embodiment, the temporary root canal sealer composition has a pH of at least 8, more preferably of at least 10 or even more preferably of at least 12. The use of a pH of at least 8 ensures that greater amounts of dispersed phase may be incorporated into the composition than if the pH of the temporary root canal sealer composition were less than this value. The pH values are measured using standard pH paper such as universal indicator paper.

The temporary root canal sealer composition comprises, based on the total weight of the composition, (e) 15 to 45 wt% of water. Preferably the temporary root canal sealer composition comprises water in an amount of from 20 to 30 wt%.

If a lower weight percent of water is employed in the temporary root canal sealer composition, the temporary root canal sealer composition has insufficient plasticity so as to be effective at sealing and maintaining the sterility of a given root canal cavity. Moreover, if a lower weight percent of water is employed in the temporary root canal sealer composition, the consistency and homogeneity of the resulting temporary root canal sealer composition is decreased, such that the composition is rendered unsuitable for use as a sealer or sterilant. The reduced plasticity, consistency and homogeneity of such composition also render it unsuitable for use as visualizing agents in combination with X-ray radiation. Conversely, if a greater weight percent of water is employed in the temporary root canal sealer composition, the remaining components thereof are effectively diluted, such that the composition takes on the characteristics of a solution. Consequently, composition comprising a greater weight percent of water is rendered too fluid to act as sealer, let alone prevent radio-opaque fillers from settling out of the composition, particularly when dense radio-opaque fillers are used in increased proportions.

In a preferred embodiment, the temporary root canal sealer composition may comprise (f) 0 to 25 wt% of a humectant. The humectant serves to keep temporary root canal sealer compositions from hardening or crystallizing upon exposure to air. The humectants comprise one or more liquids which along with water and/or other solvents make up the carrier phase in which other ingredients particularly insoluble filler particles are dispersed to provide a stable paste. An important function of humectants is to slow the temporary root canal sealer composition from drying out due to evaporation of water or other volatile materials when the package is left open or during use of product by the practitioner. Suitable humectants include edible polyhydric alcohols such as glycerin, sorbitol, xylitol, propylene glycol, butylene glycol, or liquid polyethylene glycol and liquid polypropylene glycol. Preferably, the humectant is sorbitol. Preferably sorbitol is contained in an amount of from 5 to 15 wt%. Liquid polyethylene glycol and liquid polypropylene glycol preferably have an average molecular weight of less than 1000.

Provided that the humectant is employed in the above defined general proportions, the moisture inherent to the temporary root canal sealer composition is retained during use of the temporary root canal sealer composition even if the package is left to stand in an open state for more than 6 hours. Accordingly, the temporary root canal sealer composition is effectively prevented from solidifying or becoming so viscous that the plasticity, consistency and homogeneity of the resulting temporary root canal sealer composition is decreased, thereby rendering the temporary root canal sealer composition unsuitable for use as a sealer, sterilant or visualizing material. Moreover, it is not advantageous to employ greater amounts of humectant than specified above, since this lowers the viscosity and, hence, the sealing capabilities of the temporary root canal sealer composition.

In a preferred embodiment, the temporary root canal sealer composition has a dynamic viscosity in the range of from 1 to 200 Pa·s, more preferably 10 to 60 Pa·s (at 25°C).

The dynamic viscosity is a measure of the internal resistance of a fluid to flow. In the SI system the dynamic viscosity is expressed in Pa·s. The dynamic viscosity can be measured with various types of viscometers and rheometers at a temperature of 25 °C.

In a preferred embodiment, the ratio of the total amount of components (a) and (f) to the total amount of components (b) and (c) is ((a)+(f))/((b)+(c))= 1.5 to 0.025.

In the event that the weight ratio of the total amount of components (a) and (f) to the total amount of components (b) and (c) is decreased, the excessive amounts of components (b) and (c) settle out of the temporary root canal sealer composition, thus rendering it unstable. In contrast, if the weight ratio of the total amount of components (a) and (f) to the total amount of components (b) and (c) is increased, the temporary root canal sealer composition is insufficiently radio-opaque as to be effective as a visualizing agent for use in combination with X-ray radiation.

Preferably the temporary root canal sealer composition is a one-component dental composition.

The temporary root canal sealer composition may additionally comprise further components.

The temporary root canal sealer composition may additionally contain an antiseptic. The antiseptic may be selected from antibiotics, bactericide and bacteriostatins. More specifically, the antiseptic may be selected from aqueous solutions of an alcohol or mixtures of alcohols such as ethanol, propanol and isopropanol as 60 to 90%, 60 to 70% and 70 to 80% by volume aqueous solutions, respectively; quaternary ammonium compounds such as octenidine dihydrochloride, benzalkonium chloride, cetyltrimethylammonium bromide, cetylpyridinium chloride and benzethonium chloride; phenols such as phenol, trichlorophenol, thymol, carvacrol, triclosan and chloroxylenol; iodophors such as aqueous and/or alcoholic solutions of iodine, iodoform and povidone-iodine; aqueous solutions of inorganic compounds such as sodium chloride, sodium hydroxide, sodium bisulfite, sodium hypochlorite, calcium hypochlorite and hydrogen peroxide; and chlorhexidine-based antiseptics such as chlorhexidine, chlorhexidine dihydrochloride, chlorhexidine acetate and chlorhexidine gluconate, either singly or in combination. More preferably, the antiseptic is a chlorhexidine-based antiseptic. In a preferred embodiment, the antiseptic is chlorhexidine.

The antiseptic is incorporated into the temporary root canal sealer composition in order to prevent bacterial infection of the root canal cavity from occurring either during the time that the temporary root canal sealer composition is in place, or at any point subsequent to the filling of the cavity with a permanent sealer, filling or point. In order to circumvent bacterial infection from occurring during the time in which either the temporary root canal sealer composition or a permanent sealer is in place, sufficient amounts of the antiseptic are incorporated into the temporary root canal sealer composition so as to sterilize the root canal and maintain it in this condition for the duration that the root canal is temporarily sealed, even in the event that some of the antiseptic is leached from the sealer by diffusion into the oral environment.

In order to ensure that bacterial infection has not occurred during the time that the temporary root canal sealer composition is in place, an indicator may be incorporated into the temporary root canal sealer composition. In a preferred embodiment, the temporary root canal sealer composition additionally contains a pH-indicator. The pH-indicator measures the effectiveness with which any given root canal cavity is temporarily sealed, based on a decrease in pH associated with bacterial growth in the temporarily sealed root canal cavity. As such, prior to undergoing permanent sealing the sterility of any given root canal cavity may be assessed by removing the temporary root canal sealer composition from said root canal cavity and determining its pH based on the pH-indicator incorporated therein. The pH-indicator may be selected from any of thymol blue, phenolphthalein, thymolphthalein, naphtholphthalein, alizarin yellow R, leucomalachite green and cresol red. In a preferred embodiment, the pH-indicator is thymol blue. The effectiveness with which a temporary root canal sealer composition comprising a pH-indicator has sealed and sterilized any given root canal cavity may be determined by examination of the surfaces of the temporary root canal sealer composition which were in contact with the internal surfaces of the cavity for the duration of the temporary sealing. In the case of a temporary root canal sealer comprising thymol blue, those surfaces of the dispersion which exhibit bacterial growth will have changed from blue to colorless due to localized decreases in the pH at sites of bacterial growth.

In addition to water, the temporary root canal sealer composition may additionally contain an organic solvent. The organic solvent may be selected from alcohols, esters, ethers and ketones, either singly or in combination. Preferably the solvent is a linear, branched or cyclic alkyl alcohol, or an ether or ester thereof. More preferably, the solvent is selected from methanol, ethanol, propanol, butanol, pentanol, hexanol and, where applicable, the branched or cyclic isomers thereof, and esters and ethers thereof, either singly or in combination.

The temporary root canal sealer composition may additionally comprise a strong organic base or strong organic acid, or a salt thereof for adjusting the pH of the composition. The strong organic base may be an amine base or amidine base such as an amidine resin or an amidine iatex.

The temporary root canal sealer composition may additionally comprise a filler other than the radio-opaque filler. The filler may be a finely divided particulate material which is comprised of crystalline, amorphous or metallic fibers, flakes, powders or colloids. Preferably, the filler comprises particles selected from silica gel granules, alumina granules, carbon fibers, and metallic, mineral, latex, resin, nylon, cellulose, polyester, polyolefin, polyacrylic, polyamide, polyalkylene, polyamide and polyacrylamide fibers and/or granules, either singly or in combination, which are dispersed in the aqueous dispersion medium. Preferably the filler comprises particles of silica. The filler may be present in the temporary root canal sealer composition in the form of a hydrated or non-hydrated solid. In the event that the filler is even slightly soluble in any of the other components of the temporary root canal sealer composition, the filler may also be present therein in the form of a solution.

The present invention also relates to a process for the preparation of a temporary root canal sealer comprising
(a) a step of mixing
   (i) 0.5 to 40 wt% of a water-soluble polymer having an average molecular weight of from 5000 to 100,000;
   (ii) 20 to 40 wt% of a particulate inorganic radioopaque filler;
   (iii) 0.01 to 5 wt.% of an alkali resistant particulate filler having an average particle size of from 1 to 100 nm;
   (iv) 5 to 25 wt% of an alkali earth metal hydroxide and/or alkali metal hydroxide;
   (v) 15 to 45 wt% of water; and
   (vi) 0 to 25 wt% of a humectant,
   in water for providing a mixture, and
(b) a step of dispersing the mixture.

A temporary root canal sealer is prepared (a) based on a mixture obtained by mixing the components of the composition of the present invention. The components of the temporary root canal sealer composition can be combined in any order by using common mixing and blending devices for forming an intermediate aqueous mixture. The mixing temperature is not particularly limited and usually is adjusted in a range of from 10 to 70 °C. The mixing time is not particularly limited and usually is adjusted in a range of from 3 minutes to 3 hours. The mixing operation may be performed as a batch mixing operation or as a continuous mixing operation.

The intermediate aqueous mixture obtained in step (a) is subsequently dispersed in a dispersing step. A temporary root canal sealer is prepared (b) by dispersing the mixture of the components of the temporary root canal sealer composition. Preferably, the dispersing technique may be selected from media milling, high pressure homogenizing or colloidal cone milling. According to a preferred embodiment, the dispersing technique is annular gap bead milling.

The dispersing step is used to stabilize the intermediate mixture for obtaining a highly stable composition. Solids generally must be comminuted mechanically and suitable machines such as mills are used. The term mill describes machines which perform fine size reduction. The size reduction device may be a stirrer bead mill such as an annular gap bead mill, which comprises of a mill vessel containing a moving grinding medium. By suitable dispersion technique, the solid components are broken down into particles with the required particle properties such as average size and particles size distribution. Specifically, by adjusting variable parameters, for example feeding rate, stirring rate and time, the particles size may be adjusted to that required within a narrow range.

In a preferred embodiment, an annular gap bead milling technique is used. According to the annular gap bead milling technique, the milling zone is created in the gap between a conical working vessel (stator), and a conical rotor. The gap is may be in a range of from2 to 20 mm, typically in a range of from 5 to 15, or more preferably in a range of from 6.5 to 13 mm. The movement of the rotor creates radial movement of the grinding media. The grinding media is not particularly limited and includes metal, glass or ceramic beads. Momentum amplifies the outward motion, so that the product shear force increases steadily during the milling operation. Milling beads are automatically re-introduced into the product flow as it enters the milling chamber, so that continuous circulation of the media in the milling chamber is achieved.

According to a preferred embodiment, the geometry of the grinding chamber ensures uniform particle size and distribution. Product may be fed by an external pump at the desired flow rate. Since the peripheral speed of the rotor, the width of the milling gap, the material and diameter of grinding media, media fill volume and flow velocity all affect the milling results, each of these parameters may be adjusted to create the optimum conditions for the preparation of a temporary root canal sealer composition according to the present invention.

The annular gap bead milling apparatus can be operated at a pumping speed of 13 to 31 kg/h. Preferably the gap between the conic working tank and the conic slaving body is 2 to 20 mm, preferably about 6,5 mm. The grinding beads can occupy 25-75 % of the grinding volume. Preferably the grinding beads occupy 55% of the grinding volume.

The present invention will now be described with reference to Examples which are provided by way of reference.

### Examples

### General procedure

Polyethylene glycol, water, calcium tungstate, calcium hydroxide and zirconium dioxide were weighted in a mixer and stirred until a homogeneous mixture was received. The mixer apparatus is a Linden mixer. Then this mixture was pumped into annular gap bead mill with a speed of 13 - 31 kg/h. The annular gap bead milling apparatus is a Romaco/Fryma Koruma CoBall Mill (FrymaKoruma GmbH, 79395 Neuenburg, Germany). The gap between conical working vessel and the conical rotor was 6.5 mm. The grinding beads (45 - 65 vol-% of the grinding volume) were accelerated by the movement of the conical rotor resulting in a grinding of larger particles and further homogenization of the pastes. After this processing step the paste was separated from the grinding beads by a separation unit at the product discharge unit.

**Table 1 Composition and physical properties of pastes**

| **Example** | | **1** | **2** | **3** | **4** |
|---|---|---|---|---|---|
| Raw material | | | | | |
| Polyethylene glycol ¹⁾ | % | 26.01 | 26.01 | 28.05 | 26.01 |
| Water | % | 24.99 | 24.99 | 26.95 | 24.99 |
| Calcium tungstate | % | 40.00 | 32.00 | 29.00 | 24.00 |
| Calcium hydroxide | % | 8.00 | 16.00 | 15.00 | 24.00 |
| Zirconium dioxide ²⁾ | % | 1.00 | 1.00 | 1.00 | 1.00 |
| Sum | | 100.00 | 100.00 | 100.00 | 100.00 |
| Consistency ⁵⁾ | mm | 25.7 ± 0.8 | 26.7 ± 0.8 | 29.5 ± 0.0 | 22.2 ± 0.9 |
| Extrusion force ⁵⁾ | N | 27.6 ± 1.6 | 38.2 ± 0.7 | 26.4 ± 0.9 | 60.8 ± 1.3 |
| Consistency ⁶⁾ | mm | - | - | 18 ± 0 | - |
| Extrusion force ⁶⁾ | N | - | - | 35.0 ± 0.5 | - |
| Radio opacity | mm/mm Al | - | - | 2.9 ± 0.01 | - |
| pH Value | - | - | 12.5 | 12.5 | - |

| | | | | | |
|---|---|---|---|---|---|
| 1) Polyethylene glycol Mₙ = 8000 g/mol 2) Zirconium dioxide D₅₀ = 45 nm 3) The extrusion force of a 0.75 ml syringe is measured by means of a mechanical tester, Zwick universal testing machine, using a cross-head speed of 20 mm/min. 4) The consistence and extrusion force values are measured after mixing, not after homogenization 5) measurement after mixing 6) measurement after homogenization | | | | | |

**Table 2 Stability of pastes after mixing and after homogenization**

| **Example** | **1** | **2** | **3** | **4** |
|---|---|---|---|---|
| Stability¹⁾ after mixing (a) | - | - | - | - |
| Stability¹⁾ after dispersion (b) | + | + | + | + |

| | | | | |
|---|---|---|---|---|
| 1) Stability data bases on measurement of extrusion forces of pastes that were stored at 23, 30, 37, 43, 50, 60 and 70 °C in order to investigate the paste homogeneity | | | | |

### Comparative examples

| **Product** | **Producer** | **Batch** | **pH value** | **Consist ency** | **Radio opacity** | **In-use stability^{a)}** |
|---|---|---|---|---|---|---|
| | | | - | mm | mm / mm Al | h |
| Example 3 | DENTSPLY | 1208004166 | 12.5 | 18 ± 0 | 2.9 ± 0.01 | 6.0 |
| ApexCal | Ivoclar Vivadent | P80616 | 12.9 | 21.3 ± 0.3 | 4.5 | - |
| Asphaline | Becht | BO 6560911 | 6.7 | 23.8 ± 0.6 | 2.5 ± 0.2 | - |
| Calasept | Nordiska Dental | 3-26, 2011-04 | 12.4 | 12.5 ± 0.0 | 1.0 ± 0.1 | - |
| Calcicur | VOCO | 1208525 | 12.5 | 21.5 ± 0.5 | 0.9 ± 0.1 | - |
| Calxyl | OCO-Präparate | 120103 | 12.6 | 14.0 ± 0.5 | 1.5 ± 0.1 | - |
| Hypocal | Merz Dental | 668605 | 12.5 | 17.2 ± 0.3 | 1.0 ± 0.1 | - |
| Ledermix | Riemser | 8110991 | 7.4 | 23.8 ± 0.6 | 1.0 ± 0.1 | - |
| UltraCal XS | Ultradent | B6TFZ | 12.6 | 22.7 ± 0.6 | 1.3 ± 0.2 | 0.16 |

| | | | | | | |
|---|---|---|---|---|---|---|
| a) Time in which the material is usable after the first application of the non-closed syringes with used application system. | | | | | | |

## Claims

1. A temporary root canal sealer composition, comprising based on the total weight of the composition:
(a) 0.5 to 40 wt% of a water-soluble polymer having an average molecular weight of from 5000 to 100,000;
(b) 20 to 40 wt% of a particulate inorganic radioopaque filler;
(c) 0.01 to 5 wt.% of an alkali resistant particulate filler having an average particle size of from 1 to 100 nm;
(d) 5 to 25 wt% of an alkali earth metal hydroxide and/or alkali metal hydroxide;
(e) 15 to 45 wt% of water; and
(f) 0 to 25 wt% of a humectant.

2. The temporary root canal sealer according to claim 1, wherein the water-soluble polymer is selected from polyethylene glycol, polyvinyl alcohol, and polyvinylpyrollidone.

3. The temporary root canal sealer composition according to claim 1 or 2, wherein the water soluble polymer is polyethylene glycol having an average molecular weight of from 6000 to 10,000.

4. The temporary root canal sealer composition according to any one of claims 1 to 3, which comprises 25 to 35 wt% of the water-soluble polymer.

5. The temporary root canal sealer composition according to any one of the preceding claims, wherein the particulate inorganic radioopaque filler is selected from calcium tungstate, barium sulfate, and barium tungstate, which have an average particle size of from 0.5 to 100 µm.

6. The temporary root canal sealer composition according to any one of the preceding claims, which comprises 25 to 35 wt% of calcium tungstate.

7. The temporary root canal sealer composition according to any one of the preceding claims, wherein the alkali resistant particulate filler is selected from zirconium dioxide and titanium dioxide or a mixture thereof, which have an average particle size of from 5 to 50 nm.

8. The temporary root canal sealer composition according to any one of the preceding claims, which comprises 0.5 to 2 wt.% of zirconium dioxide.

9. The temporary root canal sealer composition according to any one of the preceding claims, which comprises 10 to 20 wt.% of an alkali earth metal hydroxide or alkali metal hydroxide.

10. The temporary root canal sealer according to any one of the preceding claims, wherein the alkali earth metal hydroxide is calcium hydroxide

11. The temporary root canal sealer composition according to any one of the preceding claims, which comprises water in an amount of from 20 to 30 wt.%.

12. The temporary root canal sealer composition according to any one of the preceding claims, wherein the humectant is sorbitol.

13. The temporary root canal sealer composition according to any one of the preceding claims, wherein the humectant is contained in an amount of from 5 to 15 wt%.

14. The temporary root canal sealer composition according to any one of the preceding claims, which has a pH of at least 8.

15. The temporary root canal sealer composition according to any one of the preceding claims, which has a dynamic viscosity in the range of from 1 to 200 Pa•s (25 °C).

16. The temporary root canal sealer composition according to any one of the preceding claims, wherein the ratio of the total amount of components (a) and (f) to the total amount of components (b) and (c) is ((a)+(f))/((b)+(c))= 1.5 to 0.025.

17. A process for the preparation of a temporary root canal sealer, comprising
(a) a step of mixing:
(i) 0.5 to 40 wt% of a water-soluble polymer having an average molecular weight of from 5000 to 100,000;
(ii) 20 to 40 wt% of a particulate inorganic radioopaque filler;
(iii) 0.01 to 5 wt.% of an alkali resistant particulate filler having an average particle size of from 1 to 100 nm;
(iv) 5 to 25 wt% of an alkali earth metal hydroxide and/or alkali metal hydroxide;
(v) 15 to 45 wt% of water; and
(vi) 0 to 25 wt% of a humectant.
in water, and
(b) a step of dispersing the mixture.

18. The process according to claim 17, wherein the step of dispersing (b) is medium milling or submicron milling.

19. The process according to claims 17 and 18 wherein in the step of dispersing (b) a annular gap bead mill, a high pressure homogenizer or a colloidal conus mill is used.

## Patentansprüche

1. Temporäre Wurzelkanalfüllpastenzusammensetzung, die basierend auf dem Gesamtgewicht der Zusammensetzung umfasst:
(a) 0,5 bis 40 Gew.-% eines wasserlöslichen Polymers mit einem durchschnittlichen Molekulargewicht von 5000 bis 100.000;
(b) 20 bis 40 Gew.-% eines partikelförmigen anorganischen strahlenundurchlässigen Füllstoffs;
(c) 0,01 bis 5 Gew.-% eines alkalibeständigen partikelförmigen Füllstoffs mit einer mittleren Korngröße von 1 bis 100 nm;
(d) 5 bis 25 Gew.-% eines Erdalkalimetallhydroxids und/oder Alkalimetallhydroxids;
(e) 15 bis 45 Gew.-% Wasser; und
(f) 0 bis 25 Gew.-% eines Feuchthaltemittels.

2. Temporäre Wurzelkanalfüllpaste nach Anspruch 1, wobei das wasserlösliche Polymer aus Polyethylenglykol, Polyvinylalkohol und Polyvinylpyrrolidon ausgewählt ist.

3. Temporäre Wurzelkanalfüllpastenzusammensetzung nach Anspruch 1 oder 2, wobei das wasserlösliche Polymer Polyethylenglykol mit einem durchschnittlichen Molekulargewicht von 6000 bis 10.000 ist.

4. Temporäre Wurzelkanalfüllpastenzusammensetzung nach einem der Ansprüche 1 bis 3, die 25 bis 35 Gew.-% des wasserlöslichen Polymers umfasst.

5. Temporäre Wurzelkanalfüllpastenzusammensetzung nach einem der vorstehenden Ansprüche, wobei der partikelförmige anorganische strahlenundurchlässige Füllstoff aus Calciumwolframat, Bariumsulfat und Bariumwolframat ausgewählt ist, die eine mittlere Korngröße von 0,5 bis 100 µm aufweisen.

6. Temporäre Wurzelkanalfüllpastenzusammensetzung nach einem der vorstehenden Ansprüche, die 25 bis 35 Gew.-% Calciumwolframat umfasst.

7. Temporäre Wurzelkanalfüllpastenzusammensetzung nach einem der vorstehenden Ansprüche, wobei der alkalibeständige partikelförmige Füllstoff aus Zirkondioxid und Titandioxid oder einer Mischung davon ausgewählt ist, die eine mittlere Korngröße von 5 bis 50 nm aufweisen.

8. Temporäre Wurzelkanalfüllpastenzusammensetzung nach einem der vorstehenden Ansprüche, die 0,5 bis 2 Gew.-% Zirkondioxid umfasst.

9. Temporäre Wurzelkanalfüllpastenzusammensetzung nach einem der vorstehenden Ansprüche, die 10 bis 20 Gew.-% eines Erdalkalimetallhydroxids oder Alkalimetallhydroxids umfasst.

10. Temporäre Wurzelkanalfüllpaste nach einem der vorstehenden Ansprüche, wobei das Erdalkalimetallhydroxid Calciumhydroxid ist.

11. Temporäre Wurzelkanalfüllpastenzusammensetzung nach einem der vorstehenden Ansprüche, die Wasser in einer Menge von 20 bis 30 Gew.-% umfasst.

12. Temporäre Wurzelkanalfüllpastenzusammensetzung nach einem der vorstehenden Ansprüche, wobei das Feuchthaltemittel Sorbit ist.

13. Temporäre Wurzelkanalfüllpastenzusammensetzung nach einem der vorstehenden Ansprüche, wobei das Feuchthaltemittel in einer Menge von 5 bis 15 Gew.-% enthalten ist.

14. Temporäre Wurzelkanalfüllpastenzusammensetzung nach einem der vorstehenden Ansprüche, die einen pH-Wert von mindestens 8 aufweist.

15. Temporäre Wurzelkanalfüllpastenzusammensetzung nach einem der vorstehenden Ansprüche, die eine Zähigkeit im Bereich von 1 bis 200 Pa•s (25 °C) aufweist.

16. Temporäre Wurzelkanalfüllpastenzusammensetzung nach einem der vorstehenden Ansprüche, wobei das Verhältnis der Gesamtmenge der Komponenten (a) und (f) zur Gesamtmenge der Komponenten (b) und (c) ((a)+(f))/((b)+(c)) = 1,5 bis 0,025 ist.

17. Prozess zur Herstellung einer temporären
Wurzelkanalfüllpaste, umfassend
(a) einen Schritt des Mischens von:
(i) 0,5 bis 40 Gew.-% eines wasserlöslichen Polymers mit einem durchschnittlichen Molekulargewicht von 5000 bis 100.000;
(ii)20 bis 40 Gew.-% eines partikelförmigen anorganischen strahlenundurchlässigen Füllstoffs;
(iii) 0,01 bis 5 Gew.-% eines alkalibeständigen partikelförmigen Füllstoffs mit einer mittleren Korngröße von 1 bis 100 nm;
(iv) 5 bis 25 Gew.-% eines Erdalkalimetallhydroxids und/oder Alkalimetallhydroxids;
(v) 15 bis 45 Gew.-% Wasser; und
(vi) 0 bis 25 Gew.-% eines Feuchthaltemittels in Wasser und
(b) einen Schritt des Dispergierens der Mischung.

18. Prozess nach Anspruch 17, wobei der Schritt des Dispergierens (b) Mediummahlen oder Submikrometermahlen ist.

19. Prozess nach Anspruch 17 und 18, wobei beim Schritt des Dispergierens (b) eine Ringspaltperlmühle, ein Hochdruckhomogenisator oder eine Kolloidkonusmühle verwendet wird.

## Revendications

1. Composition d'agent colmatant de canal radiculaire temporaire, comprenant, sur la base du poids total de la composition :
(a) de 0,5 à 40 % en poids de polymère hydrosoluble ayant un poids moléculaire moyen de 5000 à 100 000 ;
(b) de 20 à 40 % en poids de charge particulaire inorganique radio-opaque ;
(c) de 0,01 à 5 % en poids de charge particulaire résistant aux alcalis ayant une taille particulaire moyenne de 1 à 100 nm ;
(d) de 5 à 25 % en poids d'hydroxyde de métal alcalino-terreux et/ou hydroxyde de métal alcalin ;
(e) de 15 à 45 % en poids d'eau ; et
(f) de 0 à 25 % en poids d'humectant.

2. Agent colmatant de canal radicalaire temporaire selon la revendication 1, dans lequel le polymère hydrosoluble est choisi parmi le polyéthylène glycol, l'alcool polyvinylique, et la polyvinylpyrollidone.

3. Composition d'agent colmatant de canal radicalaire temporaire selon la revendication 1 ou 2, dans laquelle le polymère hydrosoluble est du polyéthylène glycol ayant un poids moléculaire moyen de 6000 à 10 000.

4. Composition d'agent colmatant de canal radicalaire temporaire selon l'une quelconque des revendications 1 à 3, qui comprend de 25 à 35 % en poids de polymère hydrosoluble.

5. Composition d'agent colmatant de canal radicalaire temporaire selon l'une quelconque des revendications précédentes, dans laquelle la charge particulaire inorganique radio-opaque est choisie parmi du tungstate de calcium, du sulfate de baryum, et du tungstate de baryum, qui ont une taille particulaire moyenne de 0,5 à 100 µm.

6. Composition d'agent colmatant de canal radicalaire temporaire selon l'une quelconque des revendications précédentes, qui comprend de 25 à 35 % en poids de tungstate de calcium.

7. Composition d'agent colmatant de canal radicalaire temporaire selon l'une quelconque des revendications précédentes, dans laquelle la charge particulaire résistant aux alcalis est choisie parmi le dioxyde de zirconium et le dioxyde de titane ou un mélange de ceux-ci, qui ont une taille particulaire moyenne de 5 à 50 nm.

8. Composition d'agent colmatant de canal radicalaire temporaire selon l'une quelconque des revendications précédentes, qui comprend de 0,5 à 2 % en poids de dioxyde de zirconium.

9. Composition d'agent colmatant de canal radicalaire temporaire selon l'une quelconque des revendications précédentes, qui comprend de 10 à 20 % en poids d'hydroxyde de métal alcalino-terreux ou d'hydroxyde de métal alcalin.

10. Agent colmatant de canal radicalaire temporaire selon l'une quelconque des revendications précédentes, dans lequel l'hydroxyde de métal alcalino-terreux est de l'hydroxyde de calcium

11. Composition d'agent colmatant de canal radicalaire temporaire selon l'une quelconque des revendications précédentes, qui comprend de l'eau en une quantité de 20 à 30 % en poids.

12. Composition d'agent colmatant de canal radicalaire temporaire selon l'une quelconque des revendications précédentes, dans laquelle l'humectant est du sorbitol.

13. Composition d'agent colmatant de canal radicalaire temporaire selon l'une quelconque des revendications précédentes, dans laquelle l'humectant est contenu dans une quantité de 5 à 15 % en poids.

14. Composition d'agent colmatant de canal radicalaire temporaire selon l'une quelconque des revendications précédentes, qui a un pH d'au moins 8.

15. Composition d'agent colmatant de canal radicalaire temporaire selon l'une quelconque des revendications précédentes, qui a une viscosité dans la plage de 1 à 200 Pa•s (25 °C).

16. Composition d'agent colmatant de canal radicalaire temporaire selon l'une quelconque des revendications précédentes, dans laquelle le ratio de la quantité totale des composants (a) et (f) par rapport à la quantité totale des composés (b) et (c) est ((a)+(f))/((b)+(c))= 1,5 à 0,025.

17. Procédé de préparation d'un agent colmatant de canal radicalaire temporaire, comprenant
(a) une étape de mélange :
(i) de 0,5 à 40 % en poids de polymère hydrosoluble ayant un poids moléculaire moyen de 5000 à 100 000 ;
(ii) de 20 à 40 % en poids de charge particulaire inorganique radio-opaque ;
(iii) de 0,01 à 5 % en poids de charge particulaire résistant aux alcalis ayant une taille particulaire moyenne de 1 à 100 nm ;
(iv) de 5 à 25 % en poids d'hydroxyde de métal alcalino-terreux et/ou d'hydroxyde de métal alcalin ;
(v) de 15 à 45 % en poids d'eau ; et
(vi) de 0 à 25 % en poids d'humectant.
dans de l'eau, et
(b) une étape de dispersion du mélange.

18. Procédé selon la revendication 17, dans lequel l'étape de dispersion (b) est une mouture moyenne ou une mouture submicronique.

19. Procédé selon les revendications 17 et 18, dans lequel, dans l'étape de dispersion (b), on utilise un broyeur à billes à entrefer annulaire, un homogénéisateur haute pression ou un broyeur en cône colloïdal.
